# EUROPEAN PATENT APPLICATION

(11) **EP 4 502 165 A1**
(43) Date of publication of application: **05.02.2025**
(21) Application number: 23775365.2
(22) Date of filing: 24.03.2023
(51) Int. Cl.: C12N 15/86, C12N 7/00, C12N 5/078, C12N 5/0775

(54) **NOVEL RECOMBINANT VECTOR AND USE THEREOF**

(30) Priority: 24.03.2022 KR 20220036816
(71) Applicant: CELL&BRAIN CO., LTD., Jeonju-si, Jeollabuk-do 54871 (KR)
(72) Inventor: SUH, Hae Young, Seongnam-si Gyeonggi-do 13633 (KR); BASHYAL, Narayan, Suwon-si Gyeonggi-do 16531 (KR); LEE, Tae Young, Suwon-si Gyeonggi-do 16544 (KR); CHANG, Da Young, Yongin-si Gyeonggi-do 16936 (KR); KIM, Sung Soo, Seoul 05393 (KR); JUNG, Jin Hwa, Anyang-si Gyeonggi-do 14100 (KR)
(74) Representative: Weickmann & Weickmann PartmbB
(86) International application number: PCT/KR2023/003989
(87) International publication number: WO 2023/182870

(57) **Abstract**

The present invention relates to a recombinant vector, and a gene delivery system, recombinant virus and transformant comprising the same. The recombinant vector according to the present invention is designed to express one target gene by one promoter, and is capable of delivering two or more target genes into cells and stably expressing up to three target genes simultaneously from a single vector. Thus, this recombinant vector may be useful as an efficient gene delivery system.

## Description

### Technical Field

The present invention relates to a recombinant vector and a gene delivery system, recombinant virus and transformant comprising the same.

### Background Art

Gene therapy refers to gene-based therapeutic technology that involves introducing new genes into patient's cells, or removing genes that are malfunctioning, or replacing mutated genes with normal genes. Gene therapy products can selectively target disease-causing genes immediately after gene identification, can be prepared in a more rapid and cost-effective manner than other therapeutic antibody or compound drugs, and can increase the possibility of treating diseases that are difficult to treat with conventional therapeutic drugs.

For effective gene therapy, it is essential to first find a target gene that is important for treating the disease, and then develop a vector for delivering the gene into cells without side effects. Vectors are gene delivery vectors and may be broadly classified into viral and non-viral vectors. Viral vectors are produced by removing most or part(s) of the essential genes of a virus so that it cannot replicate on its own and inserting therapeutic genes into the virus. Although the viral vectors are capable of delivering genes into cells with high efficiency, there are problems such as difficulty in scale-up production, induction of immune response, toxicity, and emergence of replication-competent viruses, depending on the type of virus. The major viral vectors that are currently being used in the development of gene therapy products include retrovirus, lentivirus, adenovirus, adeno-associated virus, herpes simplex virus, and pox virus vectors. Meanwhile, non-viral vectors, such as liposomes and plasmids, do not induce an immune response, have low toxicity, and are easy for scale-up production, but have low gene transfer efficiency and gene expression is transient, which limits the development of gene therapy products.

In the development of gene therapy products, vector technology using adeno-associated virus or retrovirus is mainly used. Lentivirus, a type of retrovirus, has advantages in that it is able to deliver a gene regardless of cell division, the chromosomal location at which the delivered gene is inserted is distributed in a region with a relatively low risk of "oncogenic insertional mutagenesis", and the risk of tumorigenicity that can be caused by LTR (long terminal repeat) can be fundamentally blocked by deleting the U3 region in the 3'*LTR* to induce self-inactivation.

However, the size of the target gene that can be introduced into a viral vector is limited, and as the size of the target gene increases, the possibility of the target gene being lost due to homologous recombination increases. In addition, multiple target genes contained in a single vector may be silenced non-uniformly depending on the type and status of the cell, and this silencing phenomenon is difficult to predict or prevent with current technology. Therefore, it is not easy to construct a vector that uniformly expresses two or more target genes in cells (e.g., mesenchymal stem cells) without silencing, and thus studies thereon are urgently needed.

### [Prior Art Documents]

### [Patent Document]

Korean Patent No. 10-1885438

### DISCLOSURE

### Technical Problem

An object of the present invention is to provide a recombinant vector capable of delivering and stably expressing two or more target genes in a cell.

Another object of the present invention is to provide a gene delivery system comprising the recombinant vector.

Still another object of the present invention is to provide a recombinant virus comprising the recombinant vector.

Yet another object of the present invention is to provide a transformant into which the recombinant vector or the recombinant virus has been introduced.

### Technical Solution

One aspect of the present invention provides a recombinant vector comprising a constitutive transport element (CTE), a first target gene, a first promoter, a second promoter, and a second target gene, wherein the first target gene and the first promoter are in reverse orientation.

As used herein, the term "vector" refers to an expression vector capable of expressing a target gene in a suitable host cell, and means a gene construct that contains essential regulatory elements operably linked so that the inserted gene contained in the vector can be expressed. As used herein, the term "operably linked" means that a nucleic acid expression regulatory sequence that performs a general function and a nucleic acid sequence encoding a target gene are functionally linked to each other.

The vector according to the present invention may comprise expression regulatory elements such as a promoter, an operator, an initiation codon, a termination codon, a polyadenylation signal, and an enhancer, a signal sequence or a leader sequence for membrane targeting or secretion, and may be produced in various ways depending on the purpose. The promoter of the vector may be constitutive or inducible. In addition, the expression vector may comprise a selection marker for selecting a host cell containing the vector, and if the expression vector is a replication competent vector, it may comprise an origin of replication. Such vectors can self-replicate or integrate into host DNA. Examples of such vectors include plasmid vectors, cosmid vectors, bacteriophage vectors, and viral vectors.

The recombinant vector comprising the CTE, the first target gene, the first promoter, the second promoter and the second target gene may comprise two target genes and two promoters that regulate the expression of the respective target genes, thereby enabling the simultaneous expression of the two target genes from a single vector.

In addition, the recombinant vector according to the present invention may further comprise an additional target gene and a promoter that regulates the expression of the additional target gene, thereby enabling the simultaneous expression of the three genes from a single vector.

According to one embodiment of the present invention, the recombinant vector may further comprise a third promoter and a third target gene.

The "constitutive transport element (CTE)" used in the present invention refers to a factor that promotes the nuclear export of incompletely spliced mRNA, and it is known that the function of the CTE is enhanced as the CTE is located closer to the poly(A) tail, which is important for the nuclear export, translation, and stability of mRNA, thereby increasing the expression of the target gene (MR Mautino, et al., Gene Therapy. 2000. 7, 1421-1424).

As used herein, the term "target gene" refers to a gene whose expression is induced by a promoter.

The "promoter" used in the present invention is a part of DNA that is involved in binding of RNA polymerase so that transcription may be initiated. The promoter is generally located adjacent to the target gene and upstream of the target gene, and is a binding site for RNA polymerase and a transcription factor, which is a protein that guides RNA polymerase to be located at the correct transcription start site. That is, the promoter is located at the 5' end of the gene to be transcribed on the sense strand and induces RNA polymerase to bind to a corresponding site directly or via a transcription factor and initiate mRNA synthesis for the target gene. The promoter has a specific gene sequence.

The elements of the recombinant vector, i.e., the CTE, the target genes, and the promoters, may affect the expression of the target genes depending on the direction of linkage.

According to one embodiment of the present invention, the CTE may be linked to the 3' end of a poly(A) tail.

According to one embodiment of the present invention, the CTE may comprise a nucleotide sequence represented by SEQ ID NO: 1.

According to one embodiment of the present invention, the CTE may be in forward or reverse orientation.

As used herein, the term "forward orientation" means that the sequence encoded by each gene within the viral gene is in the sense orientation (5'→3'), and the "reverse orientation" means that the sequence encoded by each gene within the viral gene is in the anti-sense orientation (3'→5').

The recombinant vector according to the present invention comprises two or more promoters that induce the expression of each target gene in order to simultaneously express two or more target genes, wherein the two or more promoters may be of the same or different types.

In this recombinant vector, the second target gene and the second promoter, and/or the third target gene and the third promoter, excluding the first target gene and the first promoter that are in reverse orientation, may be in forward orientation.

According to one embodiment of the present invention, the promoter may be at least one selected from the group consisting of a Simian virus 40 (SV40) promoter, a cytomegalovirus (CMV) promoter, a minimal CMV promoter, a human ubiquitin C (UBC) promoter, a human elongation factor 1a (EF1A) promoter, a phosphoglycerate kinase 1 (PGK) promoter, and a cytomegalovirus immediate-early enhancer/chicken β-actin (CAG) promoter.

It is preferable that the first promoter, the second promoter, and the third promoter according to the present invention are different from one another in order to increase the expression of each target gene.

For example, the first promoter, the second promoter and the third promoter may be selected from the group consisting of a CAG promoter (SEQ ID NO: 2), a minimal CMV (hereinafter referred to as "mCMV") promoter (SEQ ID NO: 3), a PGK promoter (SEQ ID NO: 4) and an SV40 promoter (SEQ ID NO: 5).

More specifically, the first promoter and the second promoter may be, respectively, a CAG promoter and an mCMV promoter, an mCMV promoter and a CAG promoter, a PGK promoter and an mCMV promoter, or an mCMV promoter and a PGK promoter. Here, the first promoter is in reverse orientation.

According to one embodiment of the present invention, the target gene may be at least one selected from the group consisting of genes for disease treatment, reporter genes, selection marker genes, and cell marker genes.

The recombinant vector according to the present invention may simultaneously express two or more target genes, and thus may express each of a gene for disease treatment, a reporter gene, or a selection marker gene, or can express all of a gene for disease treatment, a reporter gene, and a selection marker gene.

As used herein, the term "gene for disease treatment" refers to a polynucleotide sequence or nucleotide sequence encoding a polypeptide that exhibits a therapeutic effect on cells that abnormally express genes, such as cancer cells.

According to one embodiment of the present invention, the gene for disease treatment may be at least one selected from the group consisting of drug-sensitizing genes, proapoptotic genes, cytostatic genes, cell growth genes, cytotoxic genes, tumor suppressor genes, antigenic genes, cytokine genes, neurogenic genes, anti-angiogenic genes, and hormone genes.

The drug-sensitizing gene is a gene that encodes an enzyme that converts a non-toxic precursor (prodrug) into a toxic substance, and it is also called a suicide gene because the cell into which the gene has been introduced dies. That is, when a precursor that is not toxic to normal cells is administered locally or systemically, the precursor may be converted into a toxic metabolite only in the target cells, thereby changing drug sensitivity and destroying the target cells. Examples of such drug-sensitizing genes include, but are not limited to, Herpes simplex virus-thymidine kinase (HSV-TK) genes that use ganciclovir or valganciclovir as a precursor, the *E. coli* cytosine deaminase gene that uses 5-fluorocytosine (5-FC) as a precursor, and the like.

The proapoptotic gene is a nucleotide sequence that, when expressed, induces programmed cell death. Examples of such proapoptotic genes include, but are not limited to, p53 gene, adenovirus E3-11.6K gene (derived from Ad2 and Ad5) or adenovirus E3-10.5K gene (derived from Ad), adenovirus E4 gene, p53 pathway genes, and genes encoding caspases.

The cytostatic gene is a nucleotide sequence that is expressed in a cell and arrests the cell cycle, and examples thereof include, but are not limited to, p21, the retinoblastoma gene, the E2F-Rb fusion protein gene, genes encoding cyclin-dependent kinase inhibitors (e.g., p16, p15, p18, and p19), and the growth arrest specific homeobox (GAX) gene.

The cell growth gene is a nucleotide sequence that promotes the division, growth, and differentiation of stem cells or already differentiated cells. Examples of such cell growth genes include, but are not limited to, hepatocyte growth factor, stem cell factor, insulin-like growth factor, epidermal growth factor, fibroblastic growth factor, nerve growth factor, transforming growth factor, platelet-derived growth factor, bone-derived growth factor, colony stimulation factor, and the like.

The cytotoxic gene is a nucleotide sequence that is expressed in a cell and exhibits a toxic effect, and examples thereof include, but are not limited to, nucleotide sequences encoding Pseudomonas exotoxin, ricin toxin, diphtheria toxin, and the like.

The tumor suppressor gene refers to a nucleotide sequence that may be expressed in a target cell and suppress a tumor phenotype or induce cell death. Examples of such tumor suppressor genes include, but are not limited to, tumor necrosis factor-α gene, p53 gene, APC gene, DPC-4/Smad4 gene, BRCA-1 gene, BRCA-2 gene, WT-1 gene, retinoblastoma gene, MMAC-1 gene, MMSC-2 gene, NF-1 gene, nasopharyngeal tumor suppressor gene located on chromosome 3p21.3, MTS1 gene, CDK4 gene, NF-1 gene, NF-2 gene, VHL gene, PD-1 (programmed death-1) gene, etc.

The antigenic gene is a nucleotide sequence that is expressed in a target cell and produces a cell surface antigenic protein that may be recognized by the immune system, and examples thereof include, but are not limited to, carcinoembryonic antigen gene and p53 gene.

The cytokine gene refers to a nucleotide sequence that is expressed in a cell and produces cytokines, examples of which include, but are not limited to, GM-CSF, interleukins (e.g., IL-1, IL-2, IL-4, IL-6, IL-12, IL-10, IL-15, IL-19, IL-20), interferons (e.g., α, β, γ), etc.

The neurogenic gene is a nucleotide sequence that promotes the formation and development of nerves, and examples thereof include, but are not limited to, brain-derived neurotrophic factor, nerve growth factor, and the like.

The neuronal differentiation gene is a nucleotide sequence involved in the differentiation of stem cells such as neuroepithelial cells into neuroblasts and neuroglia, and examples thereof include, but are not limited to, neurogenin, NeuroD, achaete-scute homolog 1 (ASCL1), and the like.

The anti-angiogenic gene refers to a nucleotide sequence that is expressed in a cell and releases anti-angiogenic factor out of the cell, and examples thereof include, but are not limited to, angiostatin, an inhibitor of vascular endothelial growth factor (VEGF), endostatin, etc.

The hormone gene refers to a nucleotide sequence that produces a hormone or its precursor, examples of which include, but are not limited to, insulin, growth hormone, thyroid stimulating hormone, adrenocorticotropic hormone, gonadotropic hormone, prolactin, luteotropic hormone, melanocyte stimulating hormone, vasopressin, and oxytocin.

In addition, any gene with therapeutic effects may be used without limitation as the gene for disease treatment.

The "reporter gene" used in the present invention is a gene that is used to monitor whether the recombinant vector has been introduced or the expression efficiency of the target gene, and any reporter gene whose expression may be monitored without damage to infected cells or tissues may be used without limitation.

According to one embodiment of the present invention, the reporter gene may be at least one selected from the group consisting of TdTomato, luciferase, copGFP isolated from *Pontellina plumata,* green fluorescent protein (GFP) isolated from *Aequorea victoria,* modified green fluorescent protein (mGFP), enhanced green fluorescent protein (eGFP), red fluorescent protein (RFP), modified red fluorescent protein (mRFP), enhanced red fluorescent protein (eRFP), blue fluorescent protein (BFP), modified blue fluorescent protein (mBFP), enhanced blue fluorescent protein (eBFP), yellow fluorescent protein (YFP), modified yellow fluorescent protein (mYFP), enhanced yellow fluorescent protein (eYFP), cyan fluorescent protein (CFP), modified cyan fluorescent protein (mCFP), and enhanced cyan fluorescent protein (eCFP) genes, without being limited thereto.

As used herein, the term "selection marker gene" refers to an antibiotic resistance gene that is used to select cells into which the gene has been introduced. Since only cells expressing the selection marker gene, that is, an antibiotic resistance gene, may survive in a medium treated with antibiotic, it is possible to select cells into which the gene has been introduced. As the selection marker gene, any antibiotic resistance gene known in the art may be used without limitation.

According to one embodiment of the present invention, the selection marker gene may be at least one selected from the group consisting of beta-lactamase, puromycin N-acteyltransferase, aminoglycoside phosphotransferase, and hygromycin B phosphotransferase genes, without being limited thereto.

As used herein, the term "cell marker gene" refers to a polynucleotide sequence or nucleotide sequence encoding a molecule such as a polypeptide, protein, lipid, glycolipid, glycoprotein, sugar, etc. that is specifically expressed on a cell membrane or cell surface.

According to one embodiment of the present invention, the cell marker gene may be at least one selected from the group consisting of sodium/iodide symporter, Thy-1 cell surface antigen (CD 90), CD3, CD4, CD8, and CD25 genes, without being limited thereto.

Such target genes may be optimized with human codons. As used herein, the term "optimized with human codons" means that when DNA is transcribed and translated into proteins in host cells, there are preferred codons depending on the host between the codons designating amino acids, which are replaced with human codons to increase the expression efficiency of amino acids or proteins encoded by the nucleic acids.

For example, the first target gene, the second target gene, and the third target gene may be selected from the group consisting of an HGF gene (SEQ ID NO: 6), an Ngn1 gene (SEQ ID NO: 7 or 8), an HSV-TK gene (SEQ ID NO: 9 or 10), and a puromycin resistance gene (SEQ ID NO: 11).

The HGF gene, a hepatocyte growth factor, is a gene that encodes a heparin-binding glycoprotein known as scatter factor or hepatopoietin-A. It is known that the HGF is produced by various mesenchymal cells, promotes cell proliferation, regulates the growth of endothelial cells and the migration of vascular smooth muscle cells, and induces angiogenesis.

The Ngn1 gene is a gene encoding Neurogenin 1, which is specifically involved in neuronal differentiation. The Neurogenin 1 plays a role in inducing neural differentiation by regulating bone-morphogenetic protein and leukemia inhibitory factor. The Ngn1 gene may comprise a nucleotide sequence represented by SEQ ID NO: 7, and if a flag is attached to the 5' end of the Ngn1 gene, the Ngn1 gene may comprise a nucleotide sequence represented by SEQ ID NO: 8.

The HSV-TK gene is a gene encoding herpes simplex virus (HSV) thymidine kinase (TK). The TK is an enzyme that catalyzes the thymidylic acid production reaction by binding the phosphate at the γ position of ATP to thymidine. It is known that the TK phosphorylates antiviral drugs such as ganciclovir, valganciclovir, acyclovir, and valacyclovir, in addition to thymidine, and the phosphorylated products induce cell death by disrupting DNA replication.

The puromycin resistance gene (PuroR) is a gene encoding puromycin N-acetyltransferase. The puromycin resistance gene inactivates puromycin, which mimics aminoacyl tRNA and interferes with translation, making it useful for selecting cells that have successfully incorporated the gene.

According to one embodiment of the present invention, at least one of the first target gene, the second target gene, and the third target gene in the recombinant vector may comprise a Kozak sequence.

The Kozak sequence is a eukaryotic translation initiation site analogous to the Shine-Dalgarno sequence in prokaryotes, and is located immediately before the codon AUG (which encodes methionine) to which ribosomes bind in the mRNA sequence.

According to one embodiment of the present invention, the Kozak sequence may comprise CCACC or CCGCC.

For example, the Kozak sequence may comprise a nucleotide sequence represented by SEQ ID NO: 12.

The recombinant vector according to the present invention is capable of regulating the expression of two or more target genes driven by two or more promoters, wherein each promoter and each target gene are operably linked to each other, so that one target gene is designed to expressed by one promoter, allowing for simultaneous expression of up to three target proteins from a single vector.

Another aspect of the present invention provides a gene delivery system comprising the recombinant vector described above.

As used herein, the term "gene delivery system" refers to a system that may increase the efficiency of delivery of a target gene and/or nucleic acid sequence into a cell, thereby increasing the expression efficiency of the target gene and/or nucleic acid sequence. Such gene delivery systems may be classified into a virus-mediated system and a non-viral system.

The virus-mediated system comprises a viral vector such as a retrovirus vector, a lentivirus vector, an adenovirus vector, an adeno-associated virus vector, a herpes simplex virus vector, or a poxvirus vector, and is known to have a higher intracellular gene delivery efficiency than a non-viral system by utilizing the inherent intracellular penetration mechanism of the virus that causes infection in eukaryotic cells such as human cells. In addition, non-viral vectors have a problem in that that, after they enter cells, the genes are degraded in endolysosomes after fusion of endosomes with lysosomes. However, viral vectors have the advantages of low gene loss and high gene transfer efficiency because they have a mechanism that bypasses lysosomes and delivers genes directly into the nucleus.

According to one embodiment of the present invention, the gene delivery system may comprise a viral vector.

More specifically, the viral vector may be derived from at least one selected from the group consisting of a retrovirus, a lentivirus, an adenovirus, an adeno-associated virus, a herpes simplex virus, and a poxvirus. Preferably, the viral vector is derived from a lentivirus.

This viral vector may be assembled into viral particles which are then introduced into cells by a transduction method such as infection.

The gene delivery system according to the present invention may perform the function of delivering the recombinant vector expressing two or more target genes into a target cell, so that two or more target genes of the recombinant vector delivered into the cell may be expressed by an intracellular transcription system.

Another aspect of the present invention provides a recombinant virus comprising the above-described recombinant vector.

According to one embodiment of the present invention, the recombinant virus may be derived from a lentivirus.

This recombinant virus may target any dividing cells, specifically immune cells or stem cells.

According to one embodiment of the present invention, the immune cells may be selected from the group consisting of neutrophils, eosinophils, basophils, macrophages, mast cells, dendritic cells, B lymphocytes, T lymphocytes, and natural killer cells, or may be derived from such cells.

According to one specific example of the present invention, the stem cell may be at least one type of cells selected from the group consisting of embryonic stem cells, fetal stem cells, adult stem cells, amniotic stem cells, cord blood stem cells, induced pluripotent stem cells, mesenchymal stem cells (MSCs), neural stem cells, hematopoietic stem cells, and cancer stem cells.

Another aspect of the present invention provides a transformant into which the above-described recombinant vector or recombinant virus has been introduced.

As used herein, the term "transformant" means a cell produced by inserting a foreign gene into a host cell, and refers to a cell transfected with the recombinant vector or transduced with the recombinant virus. The transformant is also called a recombinant host cell, a recombinant cell, or a recombinant microorganism.

As used herein, the term "host cell" refers to a cell into which the recombinant vector or the recombinant virus is to be transfected, transduced, or introduced.

The host cell may be any host cell known in the art. Examples of the host cell include, but are not limited to, NS/O myeloma cells, human 293T cells, Chinese hamster ovary cells (CHO cells), HeLa cells, human amniotic fluid-derived cells (CapT cells), COS cells, canine D17 cells, feline PG4 cells, stem cells, and the like.

The transfection can introduce a gene into a cell according to a biological, chemical or physical method known in the art. For example, the transfection may be performed by at least one method selected from the group consisting of a lipofectamine method, a microinjection method, a calcium phosphate precipitation method, an electroporation method, a liposome-mediated transfection method, a DEAE-dextran treatment method, a polybrene treatment method, a polyethyleneimine treatment method, and a gene bombardment method.

The transformant may be cultured using any medium commonly used for culturing animal cells. For example, the medium may be at least one selected from the group consisting of Eagle's MEM, α-MEM, Iscove's MEM, 199 medium, CMRL 1066, RPMI 1640, F12, F10, DMEM, a mixed medium of DMEM and F12, Way-mouth's MB752/1, McCoy's 5A, and MCDB series media.

According to one embodiment of the present invention, the transformant may be an immune cell or a mesenchymal stem cell.

The immune cells are cells that differentiate from blood stem cells and participate in immunity, and may be selected from the group consisting of neutrophils, eosinophils, basophils, macrophages, mast cells, dendritic cells, B lymphocytes, T lymphocytes, and natural killer cells, or derived from such cells.

The mesenchymal stem cells are cells that differentiate from mesenchyme into muscle cells, fat cells, osteoblasts, chondrocytes, etc. during division of the fertilized egg.

The mesenchymal stem cells are cells that maintain stemness and self-renewal ability and have the ability to differentiate into various mesenchymal tissues (plasticity). The mesenchymal stem cells may be extracted from bone marrow, adipose tissue, umbilical cord blood, synovial membrane, trabecular bone, infrapatellar fat pad, placenta, or the like. These mesenchymal stem cells have the ability to 1) suppress the activity and proliferation of T lymphocytes and B lymphocytes, 2) suppress the activity of natural killer cells, and 3) regulate the functions of dendritic cells and macrophages, and thus they may be used to treat damaged joint cartilage or nerves through allotransplantation and xenotransplantation.

More specifically, the transformant may be a mesenchymal stem cell derived from any one selected from the group consisting of bone marrow, adipose tissue, umbilical cord blood, amniotic membrane, synovial membrane, trabecular bone, and infrapatellar fat pad, without being limited thereto.

### Advantageous Effects

The recombinant vector according to the present invention is designed to express one target gene by one promoter, and is capable of delivering two or more target genes into cells and stably expressing up to three target genes simultaneously from a single vector. Thus, this recombinant vector may be useful as an efficient gene delivery system.

### Brief Description of Drawings

FIG. 1 schematically shows a series of processes including constructing a plasmid, producing a virus expressing the plasmid, and infecting target cells with the virus, according to one example of the present invention.

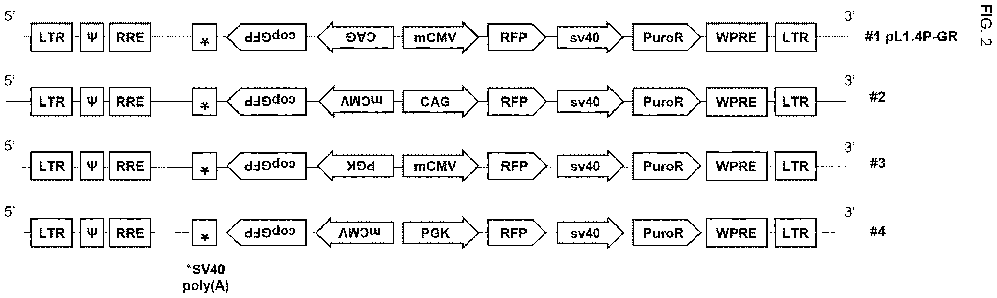

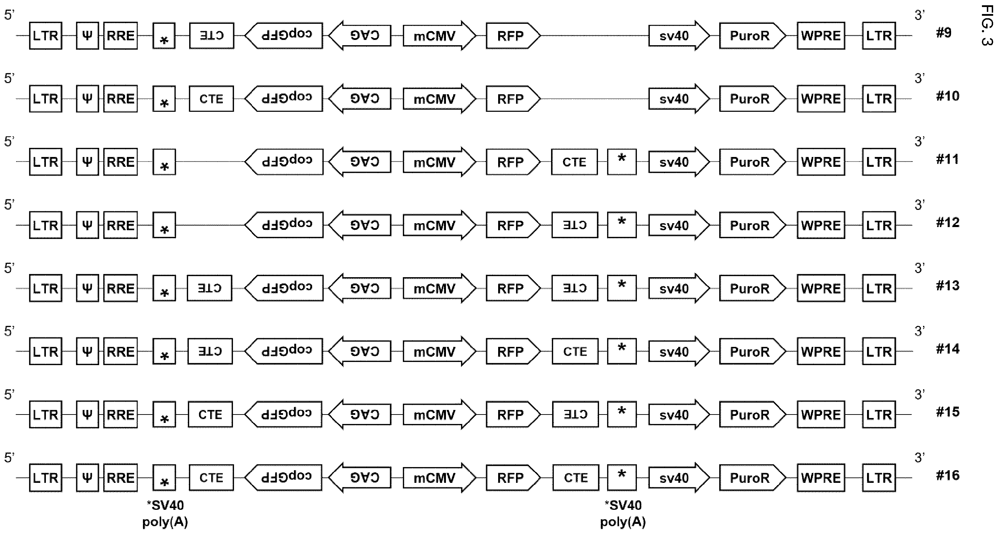

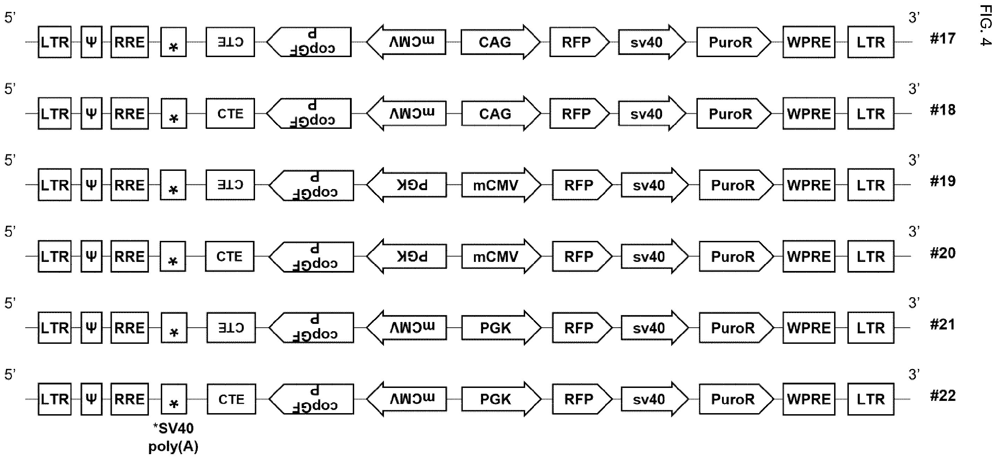

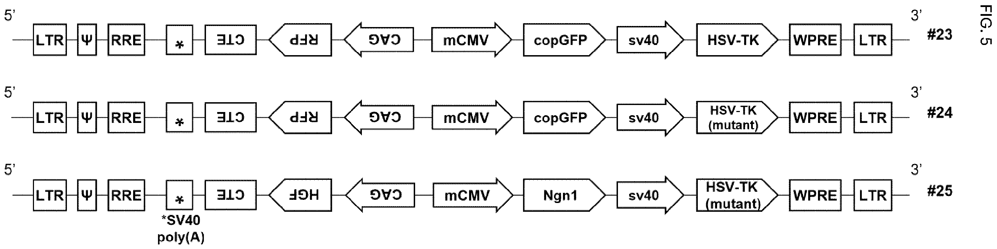

FIG. 2 shows the structures of plasmids comprising a mCMV, CAG, PGK and/or SV40 as promoters and RFP, copGFP and PuroR as target genes, constructed according to one example of the present invention. (In FIGS. 2 to 5, the mCMV promoter is oriented reversely to the CAG or PGK promoter).
FIG. 3 shows the structures of plasmids comprising mCMV, CAG and SV40 as promoters and RFP, copGFP and PuroR as target genes and comprising or not comprising CTE and SV40 poly(A), constructed according to one example of the present invention.
FIG. 4 shows the structures of plasmids comprising a mCMV, CAG, PGK and/or SV40 as promoters and RFP, copGFP and PuroR as target genes with or without CTE and SV40 poly(A), constructed according to one example of the present invention.
FIG. 5 shows the structures of plasmids comprising mCMV, CAG and SV40 as promoters, RFP, copGFP, PuroR, Ngn1, HGF and/or HSV-TK as target genes, CTE and SV40 poly(A) constructed according to one example of the present invention. (In the drawings below, the target genes are indicated in the drawings and omitted in the drawing description).
FIG. 6(A) shows the structure of pLenti-GIII-CMV-GFP-2A-Puro according to one example of the present invention, and FIG. 6(B) shows the results of comparing the virus packaging efficiency between pLenti-GIII-CMV-GFP-2A-Puro (indicated as "CMV" in the drawing) and the mCMV and CAG promoters (indicated as "CMV+CAG" in the drawing) according to one example of the present invention.
FIG. 7 shows the results of comparing the virus packaging efficiency depending on the orientation of mCMV and CAG promoters and the presence or absence of CTE according to one example of the present invention.
FIG. 8 shows the results of comparing the virus packaging efficiency depending on the orientation, location, or number of CTEs according to one example of the present invention.
FIG. 9 shows the results of comparing the virus packaging efficiency depending on the orientation of the PGK promoter and the presence or absence of CTE according to one example of the present invention.
FIG. 10 shows the results of comparing the virus packaging efficiency between the CAG promoter and the PGK promoter according to one example of the present invention.
FIG. 11 shows the results of comparing the expression rates of the target genes copGFP and RFP between the CAG promoter and the PGK promoter according to one example of the present invention.
FIG. 12 schematically shows a schedule for producing lentivirus and then infecting mesenchymal stem cells with the lentivirus, followed by cell culture, according to one example of the present invention.
FIG. 13 shows the results of comparing the persistence of expression of the target genes copGFP and RFP depending on the orientation of promoters and CTE by a fluorescence microscope, according to one example of the present invention.
FIG. 14 shows the results of comparing the persistence of expression of the target genes copGFP and RFP depending on the orientation of promoters and CTE by FACS analysis, according to one example of the present invention.
FIGS. 15 and 16 show the results of comparing the virus infection rates of vectors containing dual promoters for each cell type by FACS analysis, according to one example of the present invention. In FIG. 15, "PBNK" denotes peripheral blood natural killer cells, "Single cells" refers to a single-cell group that passed through FACS, and "Live cells" refers to living cells among the single-cell group.
FIG. 17 shows optical (BF) and fluorescent (GFP and RFP) images comparing the virus infection rate of vectors containing dual promoters for NK cells, according to one example of the present invention.
FIGS. 18 and 19 show the results of comparing the viral infection rate of vectors containing dual promoters for Jurkat T cells by FACS analysis, according to one example of the present invention.
FIG. 20 shows optical (BF) and fluorescent (GFP and RFP) images comparing the virus infection rate of vectors containing dual promoters for Jurkat T cells, according to one example of the present invention.
FIG. 21 shows fluorescence (GFP and RFP) images (A) and survival rates (B) of cells infected with a viral vector containing the suicide-inducing gene HSV-TK, measured according to one example of the present invention.
FIG. 22 shows tumor images (A) and changes in tumor volume and body weight (B) of an animal model infected with a viral vector containing the suicide-inducing gene HSV-TK, measured according to one example of the present invention.
FIG. 23 shows the results of analyzing HGF, Ngn1 and TK mRNA levels (A) and HGF expression rate (B) depending on MSC passaging, according to one example of the present invention.

### Mode for Invention

Hereinafter, the present invention will be described in more detail. However, this description is provided only as an example to help understand the present invention, and the scope of the present invention is not limited by this exemplary description.

### 1. Experimental Methods

### 1-1. Plasmid Construction

Plasmids were constructed using SV40 poly(A), CTE (SEQ ID NO: 1), promoters, including CAG (SEQ ID NO: 2), mCMV (SEQ ID NO: 3), PGK (SEQ ID NO: 4), and SV40 (SEQ ID NO: 5), target genes, including HGF (SEQ ID NO: 6), Ngn1 (SEQ ID NO: 7 or 8), HSV-TK (SEQ ID NO: 9 or 10), puromycin resistance gene (SEQ ID NO: 11), copGFP (SEQ ID NO: 13), and RFP (SEQ ID NO: 14), LTR (truncated) (SEQ ID NO: 15), Ψ (SEQ ID NO: 16), RRE (SEQ ID NO: 17), and WPRE (SEQ ID NO: 18).

For the construction of all plasmids except plasmid #1, PCR was performed under the following conditions: initial denaturation at 95°C for 5 minutes, and then 25 cycles, each consisting of denaturation at 95°C for 60 seconds, annealing at 57°C for 30 seconds, and polymerization at 72°C for 60 seconds/kb, followed by final polymerization at 72°C for 5 minutes. Digestion with restriction enzymes was performed using BamHI, FspAI, EcoRI, and PmeI, and all digestion reactions were performed at 37°C for 2 hours. Ligation reactions were performed at room temperature (RT) for 150 seconds and on ice for 10 minutes.

The primers used for plasmid construction are shown in Table 1 below.

**[Table 1]**

| Plasmid | Primer name | Primer sequence (5'→3') | SEQ ID NO. |
|---|---|---|---|
| #2 | BamHI_F | GCATGGTGGCGGATCCAGCTCTGCTTATATAGGCCT | 19 |
| | R | TGGGGCTCACCTCGACATGGTAATAGCGATGACTAATA CG | 20 |
| | F | TCGAGGTGAGCCCCACGT | 21 |
| | FspAI_R | GGAGCCCTCCATGCGCACCTTGAAGCGCATGAACTCCT TGATGACGTCCTCGGAGGAGGCCATGGTGGCCTGTAGG AAAAAGAAGAAGGC | 22 |
| #3 | BamHI_F | GCATGGTGGCGGATCCCGAAAGGCCCGGAGATGA | 23 |
| | R | ATCGCTATTACCATGGGGTAGGGGAGGCGCTTTTCC | 24 |
| | F | CATGGTAATAGCGATGACTAATACG | 25 |
| | FspAI_R | GGAGCCCTCCATGCGCACCTTGAAGCGCATGAACTCCT TGATGACGTCCTCGGAGGAGGCCATGGTGGCAGCTCTG CTTATATAGGCCT | 26 |
| #4 | R | GCGCCTCCCCTACCCCATGGTAATAGCGATGACTAATA CG | 27 |
| | F | GGGTAGGGGAGGCGCTTTTCC | 28 |
| | FspAI_R | GGAGCCCTCCATGCGCACCTTGAAGCGCATGAACTCCT TGATGACGTCCTCGGAGGAGGCCATGGTGGCCGAAAGG CCCGGAGATGA | 29 |
| #9 | EcoRI_F | CTGTCATCGAATTCCAAATCCCTCGGAAGCT | 30 |
| | R | TAACTCGAGAACATCCCACCTCCCCTGCGAGC | 31 |
| | F | GATGTTCTCGAGTTAGGCGAA | 32 |
| | EcoRI_R | CGGGCTGCAGGAATTCAC | 33 |
| #10 | EcoRI_F | GCCTGTCATCGAATTCCCACCTCCCCTGCGAGC | 34 |
| | R | TAACTCGAGAACATCCAAATCCCTCGGAAGCT | 35 |
| #11 | PmeI_F | CGTCTAGAGAGTTTCCACCTCCCCTGCGAGC | 36 |
| | PmeI_R | CACCACGCGTGTTTAACTTGTTTATTGCAGCTTATAA | 37 |
| #12 | PmeI_F | CGTCTAGAGAGTTTCAAATCCCTCGGAAGCT | 38 |
| | R | CCACCTCCCCTGCGAGC | 39 |
| | F | TCGCAGGGGAGGTGGAGATCCAGACATGATAAGATACA TTG | 40 |
| #17 | EcoRI_F | GCCTGTCATCGAATTCCAAATCCCTCGGAAGCT | 41 |
| | EcoRI _R | CGAGAACATCGAATTCCCACCTCCCCTGCGAGC | 42 |
| #18 | EcoRI_F | GCCTGTCATCGAATTCCCACCTCCCCTGCGAGC | 43 |
| | EcoRI_R | CGAGAACATCGAATTCCAAATCCCTCGGAAGCT | 44 |
| #23, 24 | HSV-TK_F | ACCGGTGCCACCATGGCTTCGTACCCCTGC | 45 |
| | HSV-TK_R | ACCGGTCTCAGTTAGCCTCCCCCATC | 46 |
| | HSV-TK_mF | CGCCATCCCATCGCCCACCTCCTGTGCTAC | 47 |
| | HSV-TK_mR | GTAGCACAGGAGGTGGGCGATGGGATGGCG | 48 |
| #25 | SV40_F | GCTAGCTCTAGAGAGTTTAAACACGCGTGGTG | 49 |
| | SV40_R | TCCGGACCAAAAAAGCCTCCTCACTAC | 50 |
| | WPRE_R | GGTACCGCGGGGAGGCGGCCCAAAGGGAGAT | 51 |
| | F | TGTACAACGTGTTTGCCTGGGCC | 52 |
| | R | CGTCGACGGTTAACGGGGATACCCCCTAGAGCCCCAG | 53 |

### Plasmid #1

The CAG-mCMV dual promoter and RFP from Mir19 tracer (Jinju Han et al., 2016, Neuron) and dscGFP (copGFP) from pGreenFier1-mCMV (Cat. No. TR010PA-P, SBI) were cloned into the Lenti-Bi-Cistronic vector (Cat. No. LV037, abm) as a backbone. Finally, plasmid #1 (pL1.4P-GR) containing reverse *CAG* and *copGFP* and forward mCMV, RFP, sv40, and puromycin resistance gene (PuroR) was constructed.

### Plasmid #2

Using plasmid #1 as the backbone, a fragment containing BamHI and FspAI in mRFP1 in forward orientation was constructed by PCR using primers. Then, a CAG-mCMV fragment was removed from a fragment constructed by enzyme digestion (BamHI and FspAI), and the two fragments were ligated together in forward orientation, thereby constructing plasmid #2 containing mCMV-CAG.

### Plasmids #3 and #4

A fragment from which *CAG* has been removed was constructed in the same manner as for constructing plasmid #2. Using the pLenti-Bi-Cistronic-CopGFP plasmid (abm) as the backbone, a fragment containing restriction enzyme sites (BamHI and FspAI), PGK, and mCMV was constructed by PCR using primers. The fragment from which *CAG* has been removed and the fragment containing PGK and mCMV were ligated together in forward or reverse orientation, thereby constructing a plasmid containing PGK-mCMV (#3) or mCMV-PGK (#4).

### Plasmids #9 and #10

Using the pLK2.4T-HN plasmid (see "plasmid #25" below) as a backbone, a fragment containing CTE and SV40 poly(A) in forward or reverse orientation was constructed by PCR using a primer containing EcoRI. Then, the constructed fragment was switched with copGFP in plasmid #1, thereby constructing a plasmid containing *SV40 poly(A)-CTE* (#9) or *SV40 poly(A)-*CTE (#10).

### Plasmids #11 and #12

Using the pLK2.4T-HN plasmid as a backbone, a fragment containing CTE and SV40 poly(A) in forward or reverse orientation was constructed by PCR using a primer containing PmeI. Then, the constructed fragment was cloned into the PmeI site of plasmid #1, thereby constructing a plasmid containing CTE-SV40 poly(A) (#11) or CTE-SV40 poly(A) (#12).

### Plasmids #13 and #14

A fragment containing CTE and SV40 poly(A) was constructed in the same manner as for constructing plasmids #11 and #12. Then, the constructed fragment was cloned into the PmeI site of plasmid #9 in forward or reverse orientation, thereby constructing plasmids #13 and #14 containing two CTEs and SV40 poly(A)

### Plasmids #15 and #16

A fragment containing CTE and SV40 poly(A) was constructed in the same manner as for constructing plasmids #11 and #12. Then, the constructed fragment was cloned into the PmeI site of plasmid #10 in forward or reverse orientation, thereby constructing plasmids #15 and #16 containing two CTEs and SV40 poly(A).

### Plasmids #17 and #18

A fragment containing CTE and SV40 poly(A) was constructed in the same manner as for constructing plasmids #9 and #10. Then, the constructed fragment was cloned into the EcoRI site of plasmid #2, thereby constructing a plasmid containing *SV40 poly (A)-CTE* (#17) or *SV40 poly(A)-CTE* (#18).

### Plasmids #19 and #20

A fragment containing CTE and SV40 poly(A) was constructed in the same manner as for constructing plasmids #9 and #10. Then, the constructed fragment was cloned into the EcoRI site of plasmid #3, thereby constructing a plasmid containing *SV40 poly(A)-CTE* (#19) or *SV40 poly(A)-CTE* (#20).

### Plasmids #21 and #22

A fragment containing CTE and SV40 poly(A) was constructed in the same manner as for constructing plasmids #9 and #10. Then, the constructed fragment was cloned into the EcoRI site of plasmid #4, thereby constructing a plasmid containing *SV40 poly (A)-CTE* (#21) or *SV40 poly(A)-CTE* (#22).

### Plasmids #23 and #24

Using the Lenti-Bi-Cistronic vector (Cat. No. LV037, abm) as a backbone, a pL2.4P-RG plasmid was first constructed by combining the CAG-mCMV dual promoter and RFP from Mir19 tracer (Jinju Han et al., 2016, Neuron) with dscGFP (copGFP) from pGreenFire1-mCMV (Cat. No. TR010PA-P, SBI), and CTEf obtained from pLVX-TetOne-Puro vector (Cat. No. 124797, Addgene).

Then, using pAL119-TK (Cat. No. 21911, addgene) as a backbone, HSV-TK was amplified by PCR, thereby constructing a fragment containing HSV-TK. After cutting the third target gene from the pL2.4P-RG plasmid by digestion with PmeI and Acc65I, the fragment containing HSV-TK (#23) or mutant HSV-TK (#24) was cloned, thereby constructing plasmids #23 and #24.

The mutant HSV-TK is one in which alanine at position 168 in the amino acid sequence of the protein is substituted with histidine, and was reported to have a kinase activity about 4 times higher than that of wild-type HSV-TK (Jan Balzarini et al., 2006, J Biol Chem.). Thus, a single amino acid mutation (A168H) was introduced by the overlapping PCR technique.

### Plasmid #25

A DNA fragment containing SV40, PuroR, and a WPRE element from the Lenti-Bi-Cistronic vector (Cat. No. LV037, abm) was obtained by PCR and cloned into a pGEM^{®}-T easy vector system (Cat. No. A1360, Promega) by TA cloning. The resulting plasmid was named pTA1. A backbone was obtained from Mir19 tracer (Jinju Han et al., 2016, Neuron) using NheI and Acc65I enzymes, and then pTA1 was digested with the same enzymes to obtain SV40 promoter, PuroR and WPRE fragments which were then cloned into the backbone plasmid. The resulting plasmid was named pCB1.

The Neurogenin1 gene was obtained by PCR using primers containing EcoRI restriction sites at both 5' and 3' ends and Flag from human cells, and was cloned into pCB1 after digestion with EcoRI. In a similar manner, the HGF gene was obtained by PCR using primers containing a NheI restriction site at the 5' end and a XbaI restriction site at the 3' end, and cloned after digestion with XbaI and NheI. The resulting plasmid was named pCB7.

*CAG*+mCMV from Mir19 tracer (Jinju Han et al., 2016, Neuron) was obtained by PCR using primers containing NheI restriction sites at both 5' and 3' ends and was cloned into pCB7 after digestion with NheI. The resulting plasmid was named pL1.3P-NH.

CTE obtained from the pLVX-TetOne-Puro vector (Cat. No. 124797, Addgene) was cloned into pL1.3P-NH, and a HSV-TK fragment was obtained from plasmid #24 by digestion with PmeI and MauBI and cloned. The resulting plasmid was named pL2.3T-NH.

pL2.3T-NH was digested with AgeI and PmeI and then re-ligated to obtain a clone with the CAG promoter cloned in reverse orientation. The obtained clone was named pLK2.4T-HN plasmid (#25).

### 1-2. Virus Packaging

Using PEI (Cat. No. 101000033, Polyplus), Lenti-X-293T cells (Cat. No. 632180, TaKaRa) (5 x 10⁵ cells/well), 0.6 µg of each plasmid constructed in Example 1 or the pLenti-GIII-CMV-GFP-2A-Puro vector (Cat. No. LV180162, abm), and 3.6 µl (1.8 µg) of the pPACKH1 HIV Lentivector packaging kit (Systembio, LV500A-1) were transfected into a 6-well plate with. After culturing in an incubator at 37°C under 5% CO₂ for 48 hours, the medium was collected, filtered through a 0.45-µm syringe filter, and immediately stored at -80°C until the next use.

### 1-3. Transduction and Virus Titration

Lenti-X 293T cells were seeded in a 24-well plate at 50,000 cells/well and cultured at 37°C under 5% CO₂ for 24 hours. After removing the culture medium, a virus dilution (2, 5, or 25-fold) obtained by dilution with DMEM containing 10% FBS and 1% P/S in a final volume of 250 µl was added to the cells in the presence of polybrene (8 µg/ml) (Cat. No. TR 1003-G, Sigma-Aldrich). The cells were cultured at 37°C under 5% CO₂ for 24 hours. The transduction medium was removed, and 500 µl of DMEM containing 10% FBS and 1% P/S was added to each well, followed by culture at 37°C under 5% CO₂ for 48 hours. After removing the medium, the cells were washed with DPBS (Cat. No. LB 001-02, WELGENE). Transduced cells were harvested using 0.25% trypsin-EDTA (Cat. No. 25200056, Thermo Fisher Scientific). Next, the cells were washed with DPBS and resuspended in eBioscience^{™} flow cytometry staining buffer (Cat. No. 00422226, Invitrogen). GFP-positive cells were sorted using the Attune NxT Acoustic Focusing Cytometer (Invitrogen) with Attune^{™} NxT software.

The virus titer was determined using the following formula: Functional titer (IFU/mL) = [(total number of cells at transduction) X (% of cells transduced) X (dilution factor)]/(volume of virus added to cells at transduction (mL)). Titer values outside the linear range were not used to determine the final titer.

For viral vectors encoding Ngn1, HGF, and HSV-TK, the percentage of transduced cells was determined by FACS analysis after intracellular HGF staining. Briefly, transduced cells were harvested using 0.25% trypsin-EDTA. Then, the cells were washed with DPBS and resuspended in 500 µl of DPBS, mixed with 500 µl of IC fixation buffer (Cat. No. 00-8222-49, Invitrogen), and then incubated for 30 minutes at room temperature. The cells were washed three times with 5 ml of 1X permeabilization buffer (Cat. No. 00-8333-56, Invitrogen) by centrifugation at 500 g at room temperature for 5 minutes each. Before treatment with secondary antibodies, the cells were blocked with blocking solution (1% albumin from BSA (Cat. No. A7906-100G, Sigma) in 1x permeabilization buffer) for 30 minutes at room temperature. After removing the blocking solution, the cells were incubated with HGF antibody (Cat. No. AB-294-NA, R&D systems) (1:200 dilution in blocking buffer) for 1 hour at room temperature. Goat IgG antibody (Cat. No. I9140, Sigma-Aldrich) (1:2000 dilution in blocking buffer) was used as a control. The cells incubated with the primary antibody were washed three times with 5 ml of 1X permeabilization buffer by centrifugation at 500 g at room temperature for 5 minutes each. Next, the cells were treated with the donkey anti-Goat IgG (H+L) cross-adsorbed secondary antibody, Alexa Fluor^{™} 568 (Cat. No. A-11057, Invitrogen) in the dark for 1 hour at room temperature. The cells treated with the secondary antibody were washed twice with 5 ml of 1X permeabilization buffer and finally with 5 ml of DPBS. The cells were resuspended in 250 µl of eBioscience^{™} flow cytometry staining buffer (Cat. No. 00-4222-26, Invitrogen). HGF positive (Alexa 568 positive) cells were measured by FACS, and viral titers were measured as previously described.

### 1-4. MSC Transduction

Mesenchymal stem cells (MSCs) were isolated from human bone marrow and passaged in MSC culture medium (DMEM (Cat. No. LM 001-05, Welgene) containing 10% FBS (Cat. No. 16000-044, Gibco), 100 U/ml penicillin and 100 µg/ml streptomycin (Cat. No. 15140-122, Gibco), and 10 ng/ml b-FGF (Cat. No. 100-18B, PeproTech)). Passage-5 MSCs were cultured in 6-well plates containing MSC culture medium at 200,000 cells/well for 24 hours. MSCs were transduced with 30 MOI of a viral vector to a final transduction volume of 1 ml in the presence of 4 µg/ml polybrene for 8 hours in a 37°C incubator supplemented with 95% O₂ + 5% CO₂. When the cells reached 80 to 90% confluency, GFP and RFP fluorescence images and optical (bright-field, BF) images were acquired using an EVOS M5000 imaging system (Thermo Fisher Scientific). Transduced cells were detached with 0.25% trypsin-EDTA and washed with DPBS. After resuspending in 1 ml DPBS, the cells were counted on a nucleocounter using Nucleoview^{™} NC-250TM software (Chemetec) according to the manufacturer's instructions. Cells were plated at 1,000 cells/cm² for the next passage. All cell culture media were replaced with fresh media every 2 to 3 days. The remaining cells were used for GFP/RFP FACS analysis as described previously.

### 1-5. In Vitro Cellular Suicide Measurement

U87 cells, a human brain cell line, were infected with lentivirus produced via plasmid #23 or #24, thus producing U87+#23 or U87+#24 cells. Each cell type was seeded in a 12-well plate at a concentration of 1 × 10⁴ cells/mL. After 24 hours of culture, the cells were treated with the required concentration of ganciclovir (GCV, Cat. No. G2536, Sigma). On day 4, the cells were treated with 0.5 mg/mL of MTT (Cat. No. M2128, Sigma). After 2 hours of incubation, each well was treated with 500 µl of DMSO to induce MTT-formazan formation. To determine the degree of cell death, the absorbance of each well at 540 nm was measured.

### 1-6. In Vivo Cellular Suicide Measurement

Animal models were generated by intraperitoneally injecting 10⁶ U87+#23 or U87+#24 cells in 100 µL of PBS containing 20% Corning^{®} Matrigel^{®} Growth Factor Deduced (GFR) Basement Membrane Matrix LDEV-free (Cat. No. 354230, Corning) into 6-week-old Balb/c nude mice. After 6 weeks, when the tumor reached an appropriate volume (100 to 300 mm³), valganciclovir (vGCV, Cat. No. V0158, Tokyo Chemical Industry) dissolved in normal saline was administered orally to the animal model daily at a concentration of 50 or 200 mg/kg for 14 days. The tumor volume was measured every other day.

### 2. Results

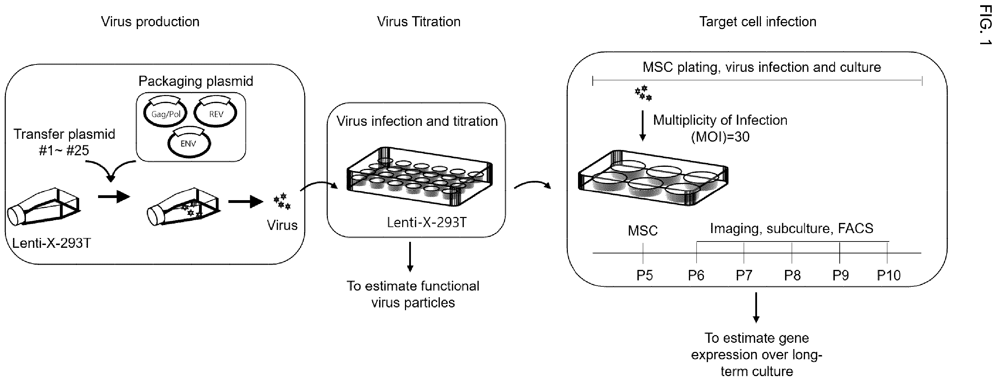

Referring to FIG. 1, the third-generation lentivirus packaging system was used, and lentivirus expressing each plasmid was produced using Lenti-X-293T (Cat. No. 632180, Takara) as a virus production cell line. The infectivity of the produced lentivirus was measured through Lenti-X-293T. Mesenchymal stem cells (MSCs) were infected with the lentivirus, and the expression rate and persistence of expression of the target gene were evaluated. The results are as follows.

### 2-1. Comparison of Virus Packaging Efficiency Depending on CAG Promoter

The lentivirus packaging efficiency was compared between the vector pLenti-GIII-CMV-GFP-2A-Puro (Cat. No. LV180162, abm), which contains the CMV promoter mainly used in conventional lentivirus production, and the vector (plasmid #1) containing the mCMV and CAG promoters.

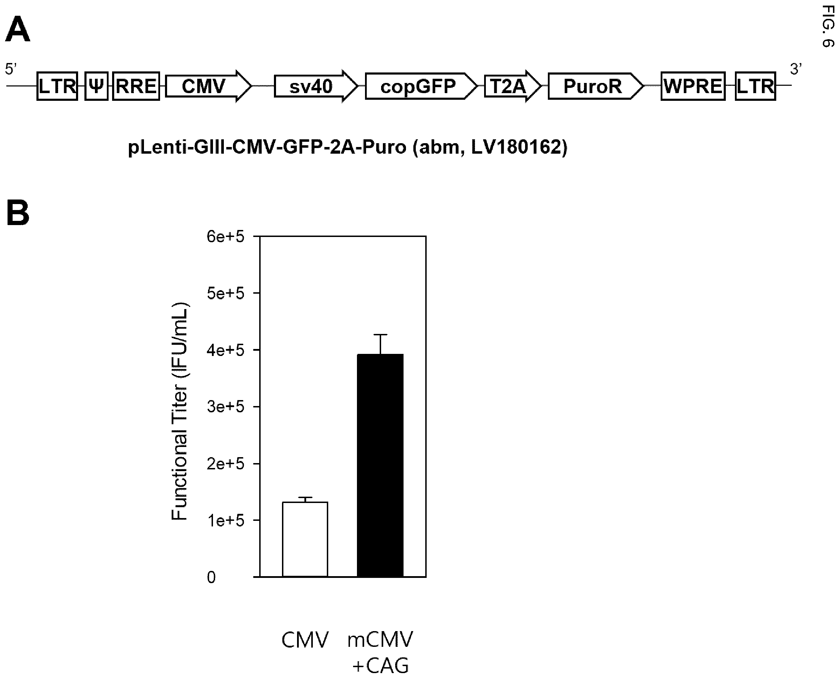

As a result, as shown in FIG. 6, it was confirmed that the mCMV+CAG vector had a higher viral titer than the vector with only the CMV promoter, and that the mCMV+CAG vector could stably perform lentivirus packaging.

### 2-2. Comparison of Virus Packaging Efficiency Depending on Orientation of CAG Promoter and Presence or Absence of CTE

Viral packaging efficiency was compared depending on the forward or reverse orientation of the CAG promoter. In addition, the effect of CTE, known to be involved in the nuclear export of RNA, on the expression of the target protein, was examined.

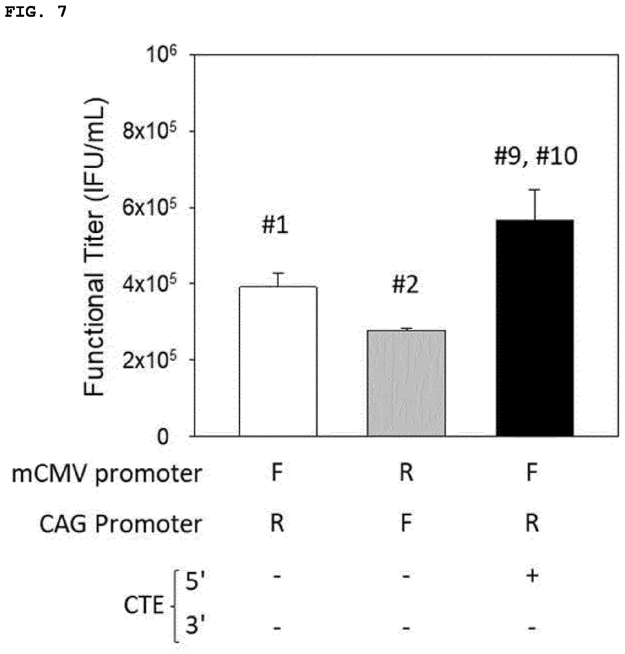

As shown in FIG. 7, when the CAG promoter was in reverse orientation (#1), the virus titer was higher than when the CAG promoter was in forward orientation (#2). In addition, the vectors containing CTE (#9 and #10) exhibited a higher virus titer.

### 2-3. Comparison of Virus Packaging Efficiency Depending on Orientation, Location, and Number of CTEs

Lentiviral packaging efficiency was compared depending on the orientation, location, and number of CTEs in vectors containing the CAG promoter positioned in reverse orientation.

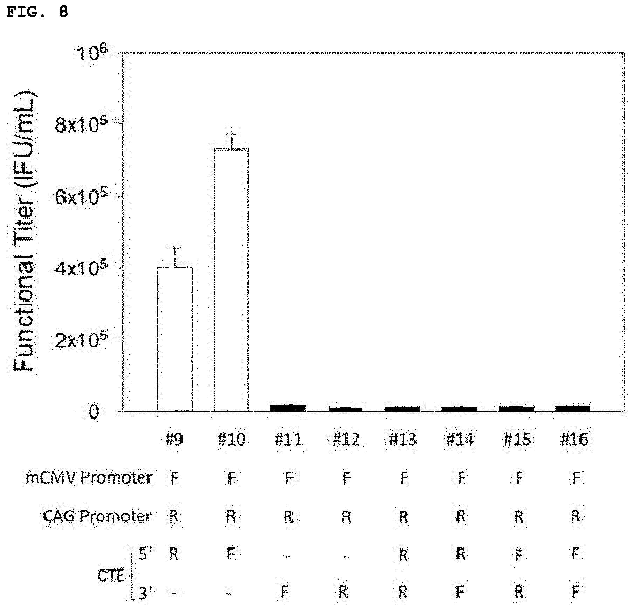

As a result, as shown in FIG. 8, when one CTE was located at the 5' end of the CAG promoter in forward or reverse orientation, the virus titer was high (#9 and #10). When the CTE was located at the 3' end of the CAG promoter, the virus was not produced properly (#11 to #16).

### 2-4. Comparison of Virus Packaging Efficiency Depending on Orientation of PGK Promoter and Presence or Absence of CTE

Virus packaging efficiency was compared depending on the orientation of the PGK promoter and the presence or absence of the CTE in vectors containing the mCMV promoter and the PGK promoter instead of the CAG promoter.

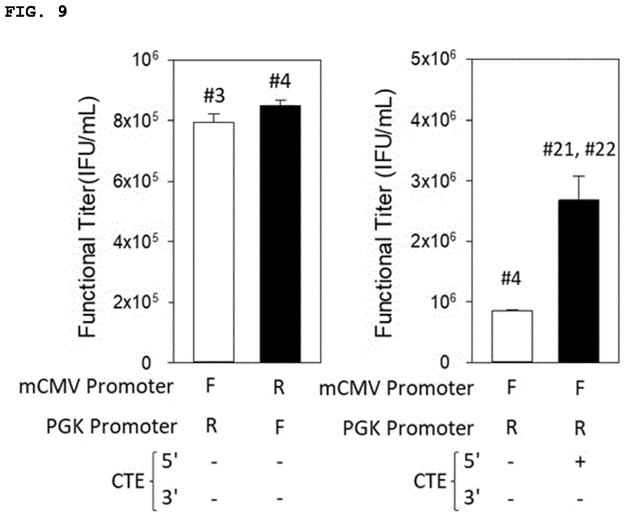

As a result, as shown in FIG. 9, the PGK promoter exhibited a constant virus titer regardless of the orientation thereof (#3 and #4), and the virus titer increased further when the CTE was present (#21 and #22).

### 2-5. Comparison of Virus Packaging Efficiency between CAG Promoter and PGK Promoter

Lentiviral packaging efficiency was compared depending on the orientation of the promoter and the CTE in vectors containing the CAG promoter and vectors containing the mCMV and PGK promoters.

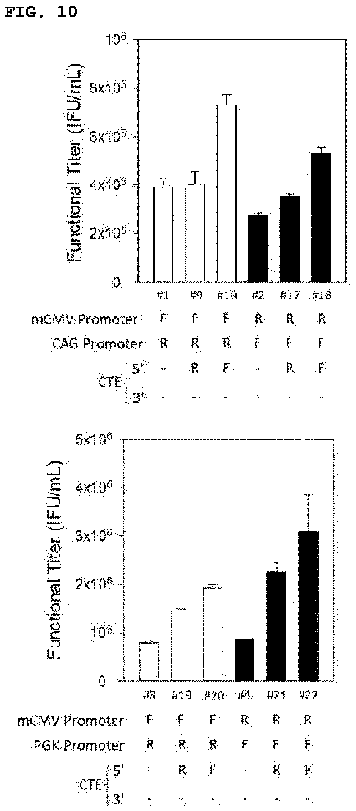

As a result, as shown in FIG. 10, regardless of the orientation of the promoter, the virus titer increased when the CTE was contained, and in particular, the virus titer increased more when the CTE was in forward orientation. When the PGK promoter was contained in forward orientation, the difference in the virus titer between the presence and absence of the CTE was up to 3 to 4 times.

### 2-6. Comparison of Expression Rates of Target Genes Depending on Orientation of CAG Promoter and PGK Promoter

To compare the expression rates of target genes between vectors containing the mCMV and CAG promoters and vectors containing the mCMV and PGK promoters, the expression rates of GFP and RFP were measured. Since promoters have a characteristic of expressing in both orientations, the same target gene has different expression rates depending on the orientation of the promoter. In the case of a vector containing the mCMV and CAG promoters, the CAG promoter portion has a strong expression rate, and in the case of a vector containing the mCMV and PGK promoters, the PGK promoter portion has a strong expression rate. The target gene downstream of these promoters is named "stronger side". In both vectors, the target gene downstream of the mCMV promoter has a weak expression rate, and this target gene is named "weaker side".

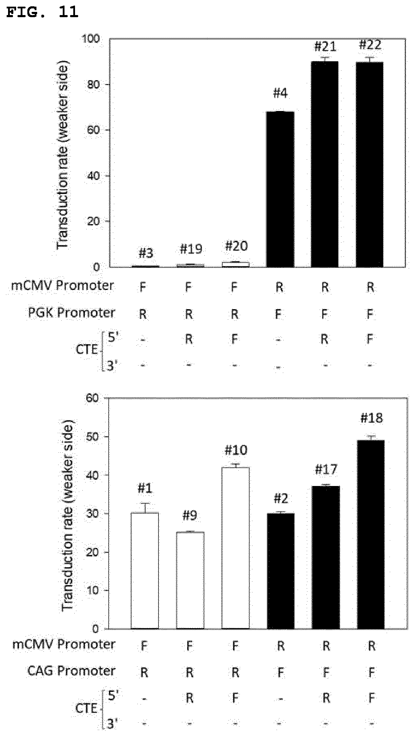

As a result, as shown in FIG. 11, in the case of the PGK promoter, the expression rate of the target gene at the weaker side differed depending on the orientation of the PGK promoter, and in particular, when the PGK promoter was in reverse orientation, the expression rate of the target gene at the weaker side was very low. However, in the case of the CAG promoter, the target gene at the weaker side was expressed equally regardless of the orientation of the CAG promoter.

### 2-7. Comparison of Expression Rate of Target Gene Depending on Orientation of Promoter and CTE

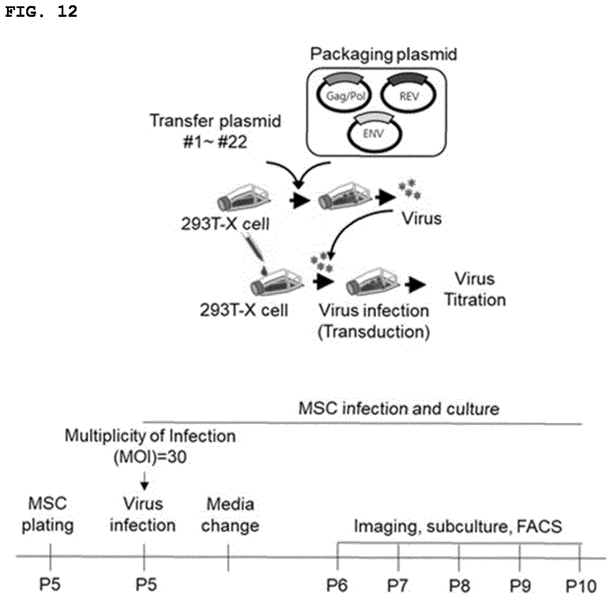

Referring to FIG. 12, after lentivirus was produced, mesenchymal stem cells (MSCs) as target cells were infected with the lentivirus. For each passage, fluorescent imaging was performed to confirm the expression of the target gene (GFP or RFP), and FACS experiments were performed to confirm the infection rate.

In addition, the persistence of expression of the target gene was compared depending on the orientation of the promoters and the CTE in a vector containing the mCMV and CAG promoters and a vector containing the mCMV and PGK promoters. In the case of the reverse CAG promoter, the stronger side was expressed as the GFP gene, and the weaker side was expressed the RFP gene. In the case of the forward mCMV+CAG promoter and mCMV+PGK promoter, the stronger side was expressed the RFP gene, and the weaker side was expressed the GFP gene.

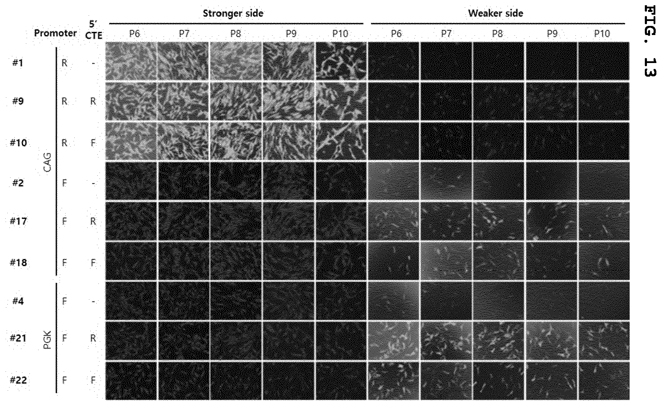

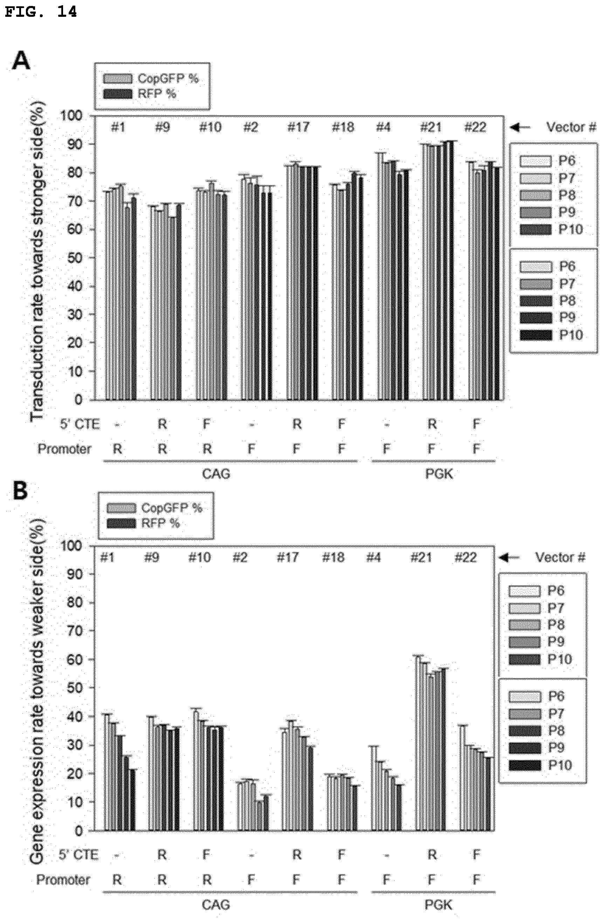

As a result, as shown in FIGS. 13 and 14, the infection rate (%) of the lentivirus was higher in the vector containing the mCMV and PGK promoters than in the vector containing mCMV and CAG. The infection rate of each vector candidate was measured with the target gene at the stronger side, and at the same time, the infection rate of the target gene at the weaker side was also measured.

The infection rates in FIGS. 14A and 14B are not the same, and even when infected with the lentivirus, when the target gene at the stronger side was expressed, the target gene at the weaker side, which is the opposite side, was not expressed in some cases. The infection rate of the target gene at the weaker side in the plasmids containing forward or reverse CTEs increased, suggesting that the disadvantages of these bidirectional promoters were overcome.

In addition, in general, the expression rate of the target gene at the weaker side of the bidirectional promoter decreased when the target cell was cultured for a long period of time (FIG. 14B, #1). The vectors containing the reverse CAG showed high persistence of target gene expression when they contained the CTE (FIG. 14B, #1 to #10), and the vectors containing the forward CAG or the vectors containing forward mCMV+PGK overcame the weaker side disadvantage only when containing the CTE in reverse orientation (FIG. 14B, #17 or #21).

These results suggest that the bidirectional plasmid candidates may show various differences between target cell types. In consideration of the difference in the expression rate of the target gene and the reduced persistence of target gene expression of expression of the target gene, which were not observed in cells such as 293T, it is necessary to select the optimal bidirectional promoter for the target cell type.

### 2-8. Comparison of Target-Gene Expression Rates of Vectors Containing mCMV+CAG or mCMV+PGK between Target Cell Types

In order to examine whether the expression rate of the target gene may differ depending on the type of target cell and whether the lentiviral plasmid candidates are applicable to various target cell types, experiments were conducted on natural killer (NK) cells and Jurkat T cells.

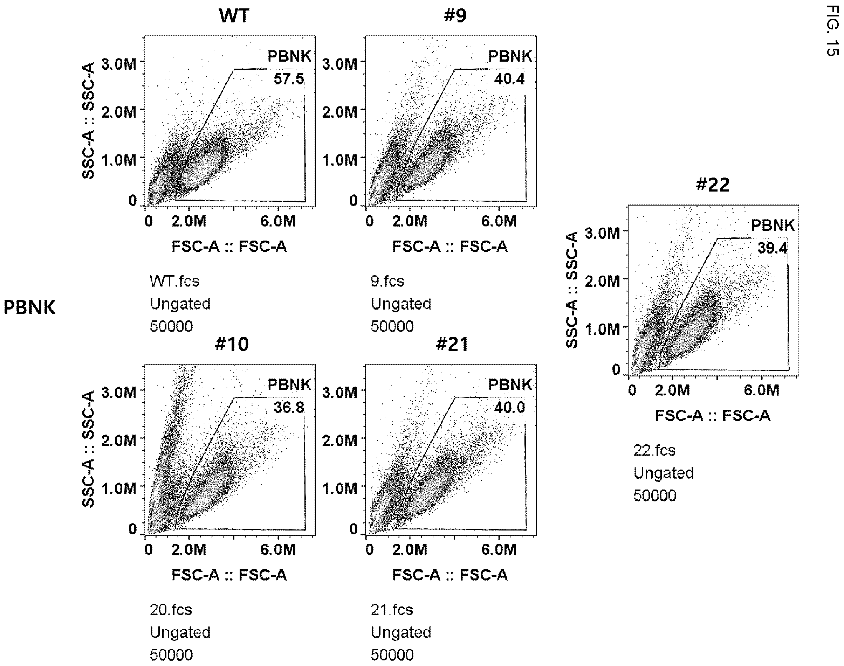

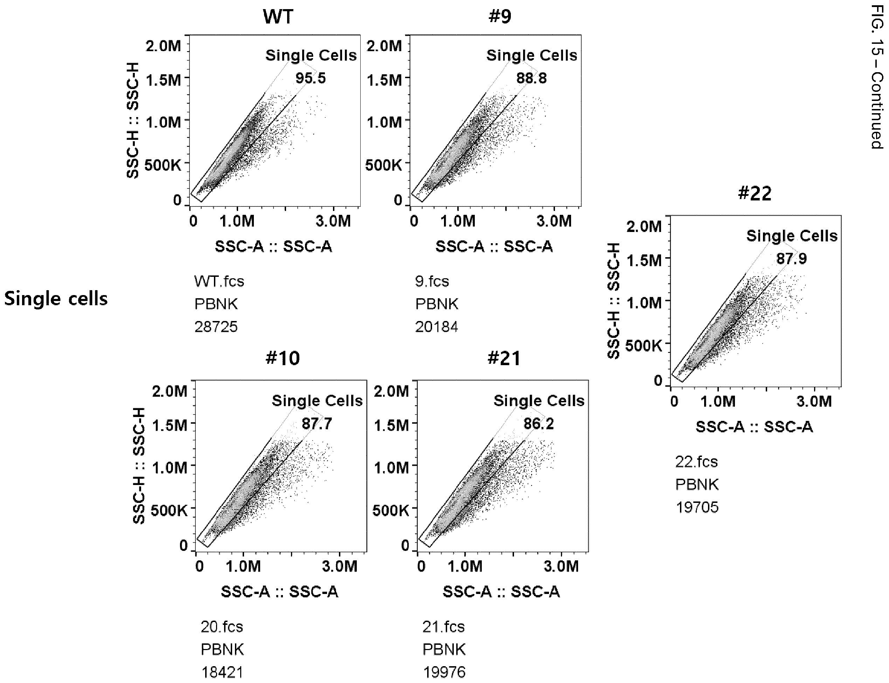

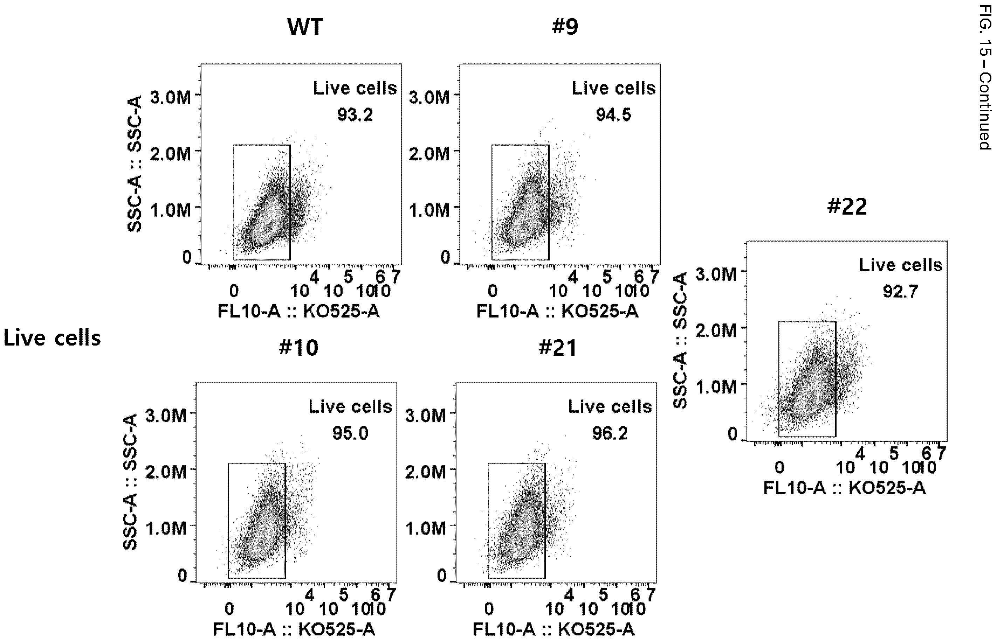

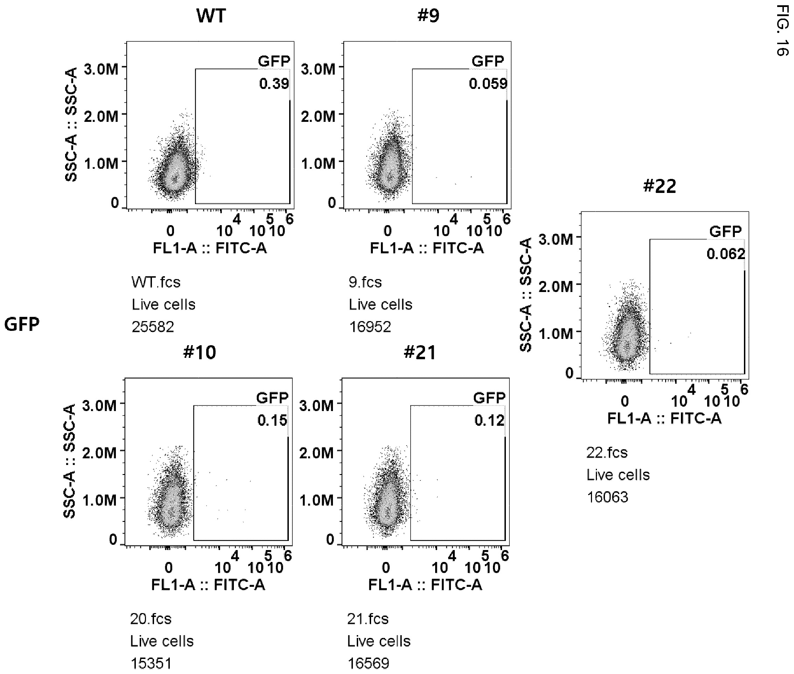

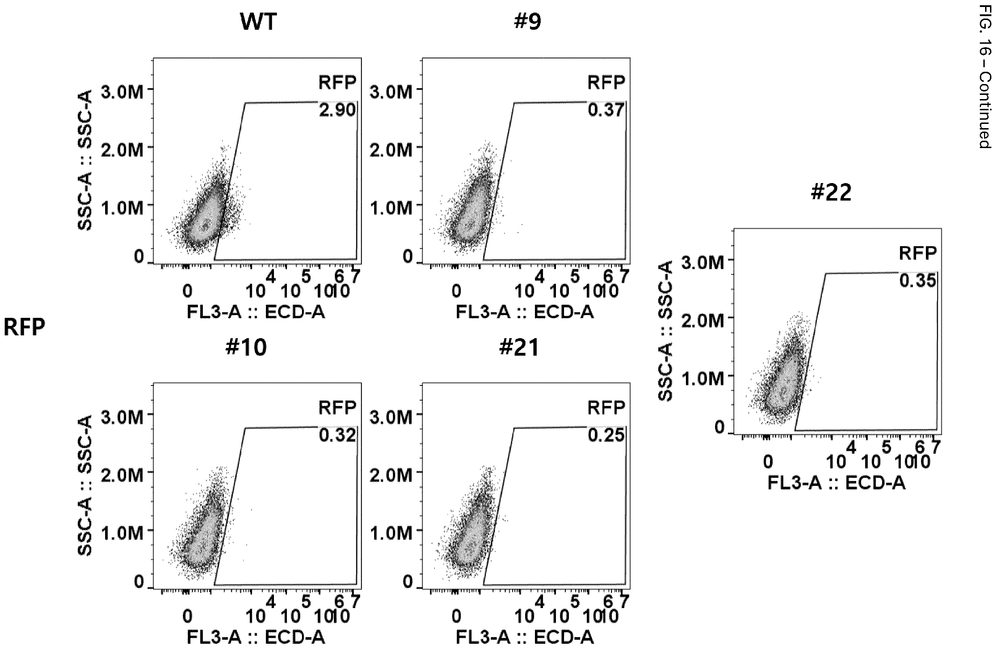

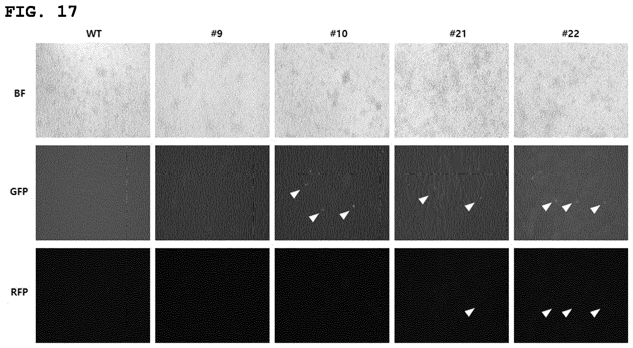

**As** a result, as shown in FIGS. 15 to 17, the infection rate of NK cells was checked by FACS and fluorescence expression rate.

When checked by fluorescence expression, more infected cells were observed in the vector containing mCMV+PGK (#21 or #22) than in the vector containing mCMV+CAG (#9 or #10). Given these results, it is expected that the use of the vector containing mCMV+PGK enables target-gene expression even in NK cells.

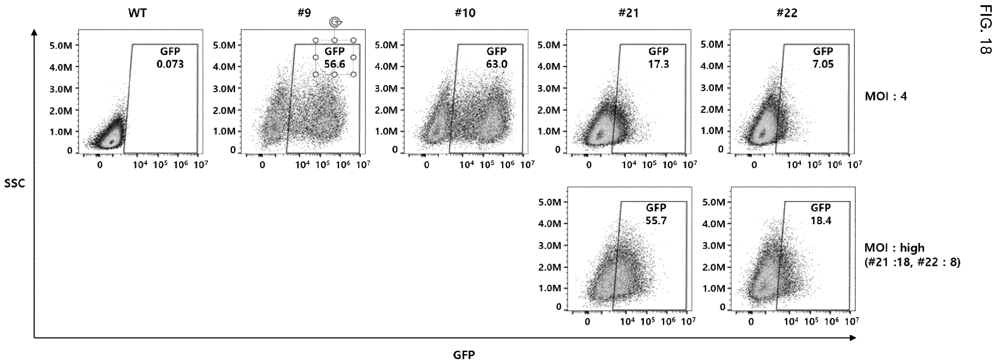

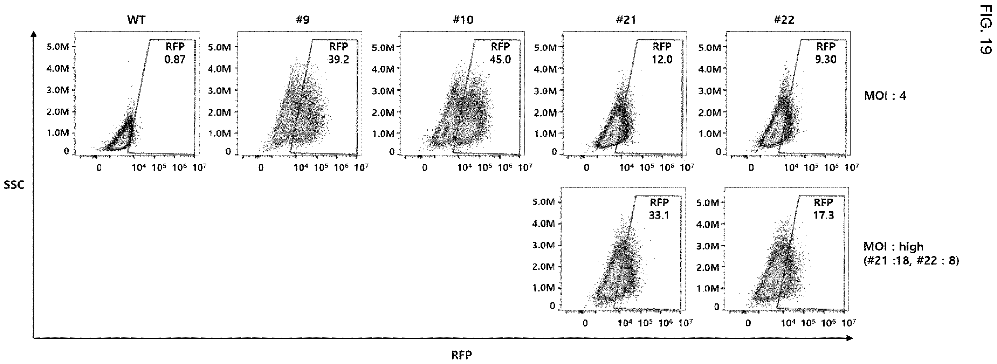

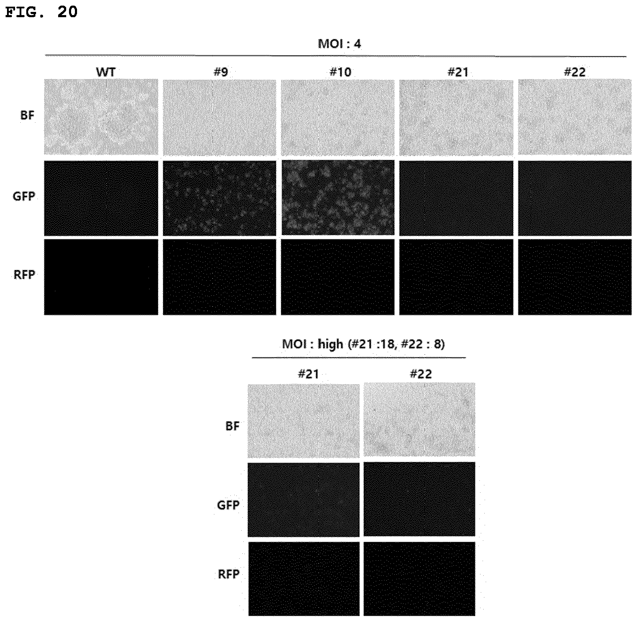

Similarly, as shown in FIGS. 18 to 20, it was found that the expression of the target gene in Jurkat T cells was higher in the case of the vector containing mCMV+CAG than in the case of the vector containing mCMV+PGK.

### 2-9. Model to Which Plasmid for Co-Expression of Target Genes Was Applied

To apply plasmids for co-expression of two or more target genes to actual research, plasmids (#23 and #24) containing a suicide-inducing gene (third target gene) were constructed, and U87 cells were transfected with the plasmids, followed by measurement of the expression rates of the genes.

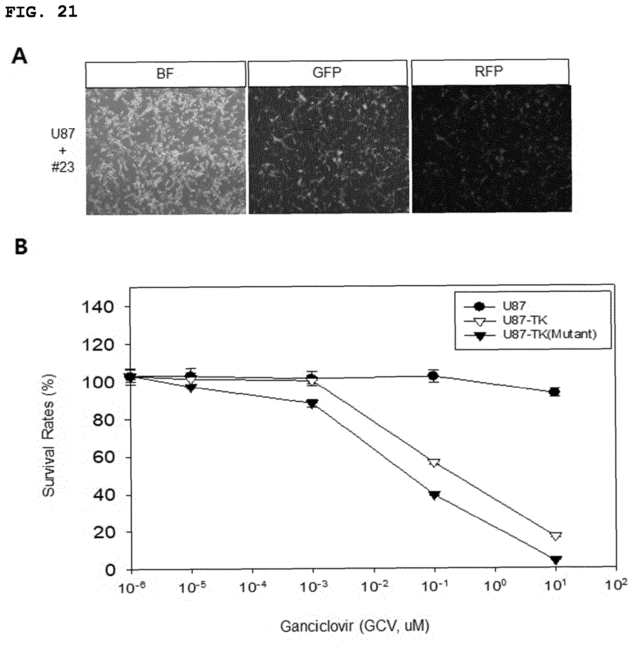

As a result, as shown in FIG. 21, normal expression of the first target gene (RFP) and the second target gene (copGFP) in the transfected U87 cells was confirmed by fluorescence, and expression of the third target gene (suicide-inducing gene, HSV-TK) in the cells was confirmed by MTT assay using ganciclovir.

A tumor formation model was generated by injecting these lentivirus-infected U87 cells into living animals, and valganciclovir was administered orally to the animal model to induce the suicide of the U87 cells.

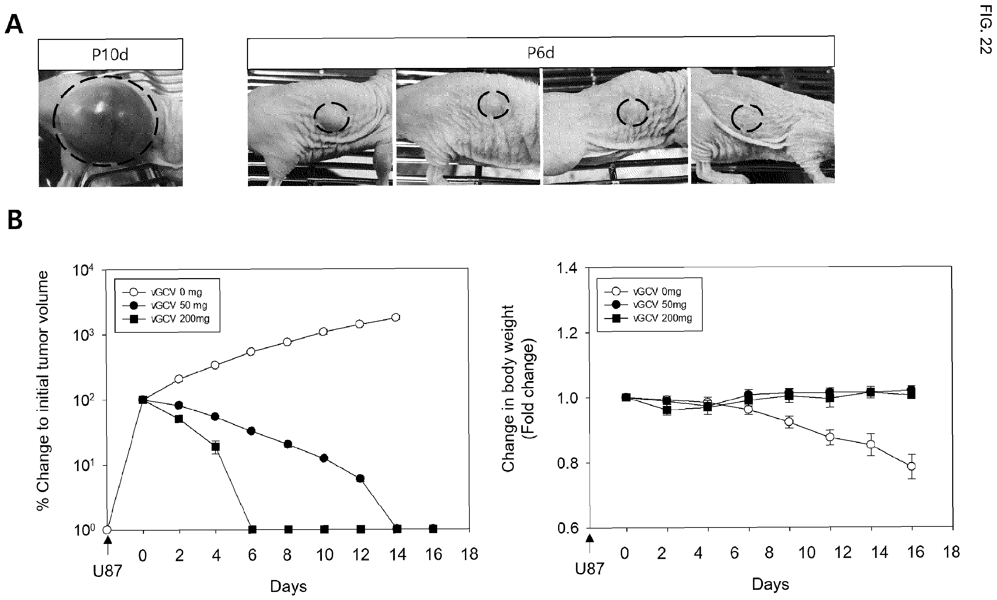

As a result, as shown in FIG. 22, it was confirmed that the tumor volume significantly decreased compared to that in the control group over time.

These results suggest that plasmids designed for co-expression of genes can be applied for experimental and industrial purposes once the target gene is replaced.

### 2-10. Confirmation of Expression after Target-Gene Change

In the previous experiment, gene expression was checked using GFP, RFP, or HSV-TK as the target gene. In this experiment, it was tested whether the expression of other target genes also occurred. To this end, after changing the first target gene to HGF, the second target gene to Neurogenin1, and the third target gene to HSV-TK, it was checked whether these genes were expressed normally.

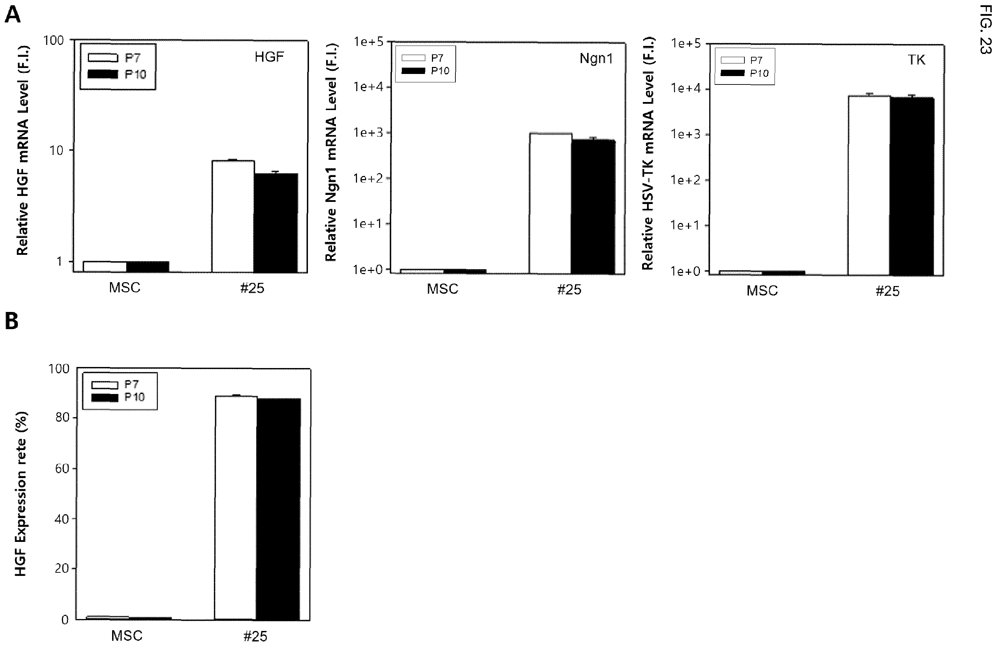

As a result, as shown in FIG. 23, it was verified that mRNA transcription occurred normally even when the target genes were changed to HGF, Ngn1, and TK, and that the decrease in expression rate with MSC passaging did not occur.

So far, the present disclosure has been described with reference to the embodiments. Those skilled in the art will appreciate that the present disclosure can be implemented in modified forms without departing from the essential features of the present disclosure. Therefore, the disclosed embodiments should be considered in descriptive sense only and not for purposes of limitation. Therefore, the scope of the present disclosure is defined not by the detailed description of the present disclosure but by the appended claims, and all modifications within a range equivalent to the scope of the appended claims should be construed as being included in the present disclosure.

## Claims

1. A recombinant vector comprising a CTE, a first target gene, a first promoter, a second promoter, and a second target gene, wherein the first target gene and the first promoter are in reverse orientation.

2. The recombinant vector of claim 1, further comprising a third promoter and a third target gene.

3. The recombinant vector of claim 1, wherein the CTE is a polynucleotide having the nucleotide sequence of SEQ ID NO: 2.

4. The recombinant vector of claim 1, wherein the CTE is in forward or reverse orientation.

5. The recombinant vector of claim 1 or 2, wherein each of the promoters is at least one selected from the group consisting of a Simian virus 40 (SV40) promoter, a cytomegalovirus (CMV) promoter, a minimal CMV promoter, a human ubiquitin C (UBC) promoter, a human elongation factor 1a (EF1A) promoter, a phosphoglycerate kinase 1 (PGK) promoter, and a cytomegalovirus immediate-early enhancer/chicken β-actin (CAG) promoter.

6. The recombinant vector of claim 1 or 2, wherein each of the target genes is at least one selected from the group consisting of a gene for disease treatment, a reporter gene, a selection marker gene, and a cell marker gene.

7. The recombinant vector of claim 6, wherein the gene for disease treatment is at least one selected from the group consisting of drug-sensitizing genes, proapoptotic genes, cytostatic genes, cell growth genes, cytotoxic genes, tumor suppressor genes, antigenic genes, cytokine genes, neurogenic genes, anti-angiogenic genes, and hormone genes.

8. The recombinant vector of claim 6, wherein the reporter gene is at least one selected from the group consisting of TdTomato, luciferase, copGFP isolated from *Pontellina plumata*, green fluorescent protein (GFP) isolated from *Aequorea victoria*, modified green fluorescent protein (mGFP), enhanced green fluorescent protein (eGFP), red fluorescent protein (RFP), modified red fluorescent protein (mRFP), enhanced red fluorescent protein (eRFP), blue fluorescent protein (BFP), modified blue fluorescent protein (mBFP), enhanced blue fluorescent protein (eBFP), yellow fluorescent protein (YFP), modified yellow fluorescent protein (mYFP), enhanced yellow fluorescent protein (eYFP), cyan fluorescent protein (CFP), modified cyan fluorescent protein (mCFP), and enhanced cyan fluorescent protein (eCFP) genes.

9. The recombinant vector of claim 6, wherein the selection marker gene is at least one selected from the group consisting of beta-lactamase, puromycin N-acteyltransferase, hygromycin B phosphotransferase, and aminoglycoside phosphotransferase genes.

10. The recombinant vector of claim 6, wherein the cell marker gene is at least one selected from the group consisting of sodium/iodide symporter, Thy-1 cell surface antigen (CD 90), CD3, CD4, CD8, and CD25 genes.

11. The recombinant vector of claim 1 or 2, wherein at least one of the first target gene, the second target gene, and the third target gene in the recombinant vector comprises a Kozak sequence.

12. A gene delivery system comprising the recombinant vector of claim 1 or 2.

13. A recombinant virus comprising the recombinant vector of claim 1 or 2.

14. The recombinant virus of claim 13, wherein the recombinant virus is derived from a lentivirus.

15. A transformant into which the recombinant vector of claim 1 or 2 or the recombinant virus of claim 13 has been introduced.

16. The transformant of claim 15, wherein the transformant is an immune cell or a mesenchymal stem cell.

17. The transformant of claim 16, wherein the immune cell is one selected from the group consisting of neutrophils, eosinophils, basophils, macrophages, mast cells, dendritic cells, B lymphocytes, T lymphocytes, and natural killer cells, or one derived therefrom.

18. The transformant of claim 16, wherein the mesenchymal stem cell is derived from any one selected from the group consisting of bone marrow, adipose tissue, umbilical cord blood, amniotic membrane, synovial membrane, trabecular bone, and infrapatellar fat pad.
